(19) Europäisches Patentamt / European Patent Office / Office européen des brevets

(11) **EP 4 284 346 B1**

(12) **EUROPEAN PATENT SPECIFICATION**

(45) Date of publication and mention of the grant of the patent:
**01.01.2025   Bulletin 2025/01**

(21) Application number: **22706695.8**

(22) Date of filing: **26.01.2022**

(51) International Patent Classification (IPC):
*A61K 9/20* (2006.01)     *A61K 31/44* (2006.01)
*G01N 33/15* (2006.01)     *G01N 33/84* (2006.01)
*G05D 21/02* (2006.01)

(52) Cooperative Patent Classification (CPC):
**G01N 33/84; G01N 33/15; G05D 21/02**

(86) International application number:
**PCT/IT2022/000005**

(87) International publication number:
**WO 2022/162709 (04.08.2022 Gazette 2022/31)**

(54) **METHOD AND DEVICE FOR SIMULATING THE EVOLUTION OF GASTRIC PH FOR IN VITRO DISSOLUTION AND RELEASE TESTS ON PHARMACEUTICAL FORMULATIONS**

VERFAHREN UND VORRICHTUNG ZUR SIMULIERUNG DES PH-VERLAUFS IM MAGEN FÜR IN-VITRO-AUFLÖSUNGS- UND FREISETZUNGSTESTS VON PHARMAZEUTISCHEN FORMULIERUNGEN

MÉTHODE ET DISPOSITIF POUR SIMULER L'HISTOIRE DU PH GASTRIQUE POUR LES ESSAIS DE DISSOLUTION ET DE LIBÉRATION IN VITRO DE FORMULATIONS PHARMACEUTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.01.2021   IT 202100001520**

(43) Date of publication of application:
**06.12.2023   Bulletin 2023/49**

(73) Proprietor: **ENHANCED SYSTEMS & TECHNOLOGIES S.R.L.**
**83100 Avellino (IT)**

(72) Inventors:
• **LAMBERTI, Gaetano**
  **83100 Avellino (IT)**
• **IANNONE, Marco**
  **83100 Avellino (IT)**
• **CACCAVO, Diego**
  **83100 Avellino (IT)**

(74) Representative: **Masetti Zannini de Concina, Alessandro**
**Akran Intellectual Property**
**Via del Tritone, 169**
**00187 Roma (IT)**

(56) References cited:
**US-A- 4 867 192          US-A- 5 643 797**
**US-A1- 2011 165 127**

• **CASCONE SARA ET AL: "In Vitro Simulation of Human Digestion: Chemical and Mechanical Behavior", DISSOLUTION TECHNOLOGIES, vol. 23, no. 4, 1 January 2016 (2016-01-01), US, pages 16 - 23, XP055850250, ISSN: 1521-298X, DOI: 10.14227/DT230416P16**
• **SARA CASCONE ET AL: "The influence of dissolution conditions on the drug ADME phenomena", EUROPEAN JOURNAL OF PHARMACEUTICS AND BIOPHARMACEUTICS, ELSEVIER SCIENCE PUBLISHERS B.V., AMSTERDAM, NL, vol. 79, no. 2, 8 April 2011 (2011-04-08), pages 382 - 391, XP028391557, ISSN: 0939-6411, [retrieved on 20110416], DOI: 10.1016/J.EJPB.2011.04.003**

**Description**

**Field of the invention**

The present invention relates to a method and a device for simulating the evolution of gastric pH, which allows to carry out *in vitro* dissolution and release tests on pharmaceutical formulations. More specifically, the invention concerns a device for reproducing predetermined temporal trends of pH in a controlled environment, the device being able to simulate the changes that this variable undergoes along the gastrointestinal tract, or to create other arbitrary pH "histories". The device is particularly suitable for simulating the different pH profiles during digestion, to make the dissolution and release analysis of pharmaceutical formulations for oral administration more accurate.

**Background of the invention**

The control of pH in chemical processes suffers from the well-known problem of non-linearity, but in the processes of simulating the behavior of physiological environments it becomes all the more important the more in-depth the level of analysis to be conducted is. In particular, when it comes to dissolution systems that aim at reproducing the conditions of the gastrointestinal tract, the control of pH becomes fundamental, because this variable greatly influences the fate of many compounds used in the pharmaceutical field.

It is well known that organs such as the stomach and the intestine, while being part of a single path, operate at completely different pH and that, moreover, the conditions of the individual organ may vary from time to time. An example of this is given by the different values that the pH assumes over time inside the stomach, in fasting conditions or during digestion.

Studies conducted using intelligent sensing devices, such as Medtronic's Smartpills™, a gut motility assessment system based on an ingestible capsule that measures pH, temperature, pressure and transit time through the entire gastrointestinal tract, show how the gastric pH value changes over time if the individual ingests food. Specifically, it has been found that when a meal is eaten, the stomach pH changes from an initial value of about 5 to a pH of about 1.5, through a sigmoidal trend (Dressman, J.B. et al., Upper Gastrointestinal (GI) pH in Young, Healthy Men and Women, Pharm. Res. 1990, 7 (7): 756-761).

Despite the knowledge of this, the dissolution and release tests of the pharmaceutical dosage units, to date, are conducted following the rules suggested by the pharmacopoeias. These provide, depending on the characteristics of the formulation to be analyzed, which devices and working conditions will have to be used for the tests, but provide, in the simulation of the gastric permanence of the dosage unit, a fixed pH value for all the different formulations.

An example is given by the extended release formulations: in this case the suggestions are to carry out the tests inside standardized equipment (e.g. USP-II), and the suggested working conditions are to carry out two isothermal steps (T $=$ 37 $\pm$ 0.5 °C), the first using 750 mL of a 0.1 N solution of hydrochloric acid in distilled water (pH ~ 1.5) as dissolution medium for two hours (average residence time of the bolus in the stomach), and the second, which begins at the end of the two hours (time that marks the passage of the chyme from the stomach to the intestine), at pH ~ 6.8. This last pH level is reached by adding solutions of sodium hydroxide or tribasic sodium phosphate dodecahydrate in distilled water, and is maintained for a time of at least 45 minutes (The United States Pharmacopeial Convention (USP), Harmonization methods, 2011, hiips://www usp org/sites/default/files/usp/documen t/har-monization/gen-method/stage_6_monograph_25_feb_2011.pdf).

In the past years, various unconventional dissolution apparatuses, or dynamic gastrointestinal simulation systems, have been created, aimed at taking into account the differences between reality and the tests suggested by the pharmacopoeias. Apparatuses which, in summary, could give more scientific evidence on the performance of the pharmaceutical formulations under examination before proceeding with the *in vivo* experiments.

These devices are often aimed at the realistic simulation of food digestion, starting from the transformations due to the amylase of saliva up to those due to the bile juices secreted in the intestine, and therefore they are already originally designated for research in the food field. Not infrequently, in fact, in addition to the use of foods of different nature and composition, the dissolution media used are complex fluids that are difficult to have available (SGF - Simulated Gastric Fluids), the dosage and use of which require the intervention of specialized operators. Many of these systems, for simplicity, approximate the stomach pH to a fixed value averaged over all the values that this variable assumes in the gastric residence time: this is the case of those devices based on the INFOGEST protocol, which provide for a constant value (equal to pH = 3) during the gastric phase (Mackie, A. and Rigby, N., InfoGest consensus method, in The Impact of Food Bioactives on Health, Springer, Cham (CH), 2015, p. 13-22; Brodkorb, A. et al., INFOGEST static in vitro simulation of gastrointestinal food digestion, Nature Protocols 2019, 14 (4): 991-1014).

In an extension of the INFOGEST protocol, a recently presented method (Mulet-Cabero, A.-I. et al., A standardized semi-dynamic in vitro digestion method suitable for food - an international consensus, Food Funct. 2020, 11 (2):1702-1720) is able to simulate a pH evolution comparable to that recorded *in vivo* after food ingestion. The pH, assumed to be unitary in the baseline condition, is increased following the introduction of food (and fluid simulating saliva), and is then again slowly brought back close to the baseline condition (pH = 2) by adding HCl 1 M in a quantity known and established by previous tests. This method shows various difficulties of application, including the absence of control and the need to determine the amount of acid to add by means of preliminary experiments.

The method described above is quite similar to that already proposed by a 1988 article (Vatier, J. et al., A model of an 'artificial stomach' for assessing the characteristics of an antacid, Aliment. Pharmacol. Therap., 1988, 2 (5):461-470): after an increase in pH from the baseline condition (in this case by adding aluminum phosphate), gastric juices or a 0.1 N solution of HCl were added at a constant rate, obtaining pH values with a decreasing sigmoidal trend, similar to those recorded in vivo.

Similar behavior have apparatuses such as HGS, Human Gastric Simulator (Kong, F. and Singh, R.P., A human gastric simulator (HGS) to study food digestion in human stomach, J. Food Sci. 2010, 75(9): E627-E635), which represents one of the first models to have reproduced the fluid dynamics of the stomach avoiding to oversimplify it, unlike the previously designed devices. The main components of this system are: a chamber with a latex coating to emulate the stomach; a mechanical system consisting of 12 rollers fixed on belts operated by a motor unit, which pushes the 'gastric walls'; systems for gastric secretion, for emptying and lastly for temperature control. The gastric secretion is simulated with a peristaltic pump which delivers the simulated gastric juice into the stomach through a tube, which in turn divides into 5 other tubes placed at different heights in the latex chamber, to ensure a homogeneous distribution of the secretions. The flow rate through the main tube is adjusted by means of a valve, and the realization of the pH profile is effected by feeding a constant flow rate of gastric juices. These latter are composed of pepsin, gastric mucin and NaCl in 1 L of distilled water, to give a pH of 1.3, which is adjusted with 6 N solutions of HCl. Also in this case, the quantity of acids and gastric juices to be introduced to obtain the desired pH profile must be decided upstream, following preliminary experiments.

The Chinese patent CN 108364553B (University of Jiangnan), having title "Human body stomach-small intestine digestion simulation method and deviation", published in 2018, describes an experimental device similar to the last three described above, capable of simulating, in two subsequent sections, both the gastric environment and the environment of the small intestine, connected in sequence, during digestion. Also in this case, the device makes use of the calibrated and timed introduction, in the two environments that simulate the stomach and small intestine respectively, of simulated gastric juices on the one hand and of simulated pancreatic and biliary juices on the other, which are dosed in predetermined quantities to obtain the predetermined pH profiles.

Slightly more complex devices, capable of controlling an acid solution dosing pump, require the use of a feedback type control. In these cases, once a certain reference pH value (set-point), constant or a function of time, is known, it is possible, through the use of a pH probe, a data acquisition device and a calculator, to quantify the distance of the pH value read from the expected one, and control the dosing pump appropriately.

The DGM Dynamic Gastric Model belongs to this class of models, designed to investigate the effects of biochemical and physical processing of foods and drugs for oral administration (Thuenemann, E.C. et al., Dynamic gastric model (DGM), in The Impact of Food Bioactives on Health, Springer, Cham (CH), 2015, p. 47-59). The aim of the research group was to define together the physical and biochemical characteristics of the human stomach with data regarding gastric residence times and emptying profiles, and to design a mechanical stimulation, controlled by a PC, which works in real time on realistically chewed meals, or on oral medicaments or nutraceuticals.

The DGM has several functions: it is capable of analyzing soft drinks, chewed meals of normal size, drugs for oral administration; it operates at 37°C with heat transfer rates similar to the *in vivo* ones; it creates moderate mixes (0.05 Hz) in the main body of the 'stomach'; it has flow rates of acids, salts, and enzymes similar to the physiological ones added around the gastric bolus; it controls and measures the addition of acid and enzymes through the automatic regulation of a pump, depending on the food present and the conditions of the lumen, controlling through feedback loop methods. The addition of acids and enzymes takes place through perforated circles located on the walls of the model. The pH inside the main body is monitored by a series of flexible 'naso-gastric' electrodes which provide feedback for the control of the pumps.

Also in the field of simulating the behavior of the gastrointestinal apparatus, the TIM (Gastro-Intestinal Model) of TNO Triskelion (Minekus, M., The TNO gastro-intestinal model (TIM), in The Impact of Food Bioactives on Health, Cham (CH), 2015, p. 37-46) is a multi-compartmental model designed since the 1990s, conceived and built to realistically simulate the anatomical and physiological characteristics of the gastrointestinal tract. The system consists of different parts, each representing a section of the gastrointestinal tract, and is made through the use of bags of elastic material immersed in thermostatic baths contained in rigid jackets, which have the purpose of simulating gastric motility and simultaneously of ensure that the temperature is constant and equal to 37°C. In this device, pH is measured by sensors placed in various points of the apparatus, and controlled through a feedback loop that ensures the regulation of the flow rate to be delivered to a pump that takes a hydrochloric acid solution. The control can be achieved by following a predetermined curve and with the aid of a computer.

Similarly, another group of researchers (Ménard, O., et al., Validation of a new in vitro dynamic system to simulate infant digestion, Food Chem. 2014, 145:1039-1045), in the multi-compartment model proposed, simulating the gastrointestinal tract of newborns, uses a feedback control allowing the regulation of the flow rate of a pump taking simulated gastric juices and delivering them in such a way as to follow a decreasing trend in pH. Also in this case, the device includes a pH probe and a pump, and the control takes place with the aid of a computer.

In the artificial stomach model proposed by Cascone et al., which is designed, unlike the models described so far, to be a non-conventional device for the dissolution of drugs rather than a device not for simulating digestion (Cascone, S., et al., In vitro simulation of human digestion: chemical and mechanical behavior, Dissolution Technol. 2016, 23(4):16-23), a pH feedback control system capable of following a set-point variable over time, and able to simulate pH "histories" comparable to real physiological conditions is implemented. In this case, the system consists of a latex bag placed vertically and wrapped in bands, which constrictions purport to simulate gastric peristalsis. The device includes a pH probe connected to an acquisition board which, through the aid of a computer, adjusts the flow rate delivered respectively by one of two pumps, one with an acidic solution and the other with a basic solution. In this way it is theoretically possible to accurately simulate the decrease in pH which occurs during digestion, also counteracting any alkalization reactions due to the pharmaceutical form in use. This therefore allows a greater degree of flexibility to be achieved with respect to the models previously described.

In the latter device, therefore, a decrease in pH is achieved in an artificial stomach model, a behavior produced by adding two solutions, one with an acid reaction and one with an alkaline reaction, on the basis of the pH value measured by a probe.

All the methods which are part of the state of the art focus attention on the *in vitro* simulation of the stomach in terms of mechanical, chemical and thermal conditions. Although this approach is scientifically remarkable, the industrial implications are less interesting, since such devices are not very versatile, difficult to reproduce and economically not feasible for standardized dissolution and drug release tests.

In the light of the aforementioned prior art, the present invention has the purpose, leaving aside the simulation of the mechanical stresses of peristalsis, to develop a method and a simple and economical device, operating autonomously as a stand-alone device, capable of automatically reproducing the *in vitro* simulation of physiological pH conditions occurring in the gastrointestinal tract, and which may be useful in particular for dissolution and release tests of pharmaceutical formulations for oral administration, to be also applied on standardized systems (for example USP II).

## Summary of the invention

In order to improve the conventional drug release and dissolution tests, a method and a device have been developed, according to the present invention, which are capable of simulating and faithfully reproducing the pH changes occurring over time in the gastric environment, represented by a buffer solution, used to test pharmaceutical dosage units for oral administration.

The proposed method exploits two simple solutions (acid and base), compatible with the pharmacopoeial standards, and a stand-alone device capable of performing a feedback control on the pH of the solution. Specifically, in the proposed method the dissolution medium is represented by the common saline solutions suggested by the various pharmacopoeias, such as for example PBS - Phosphate Buffer Saline. Thanks to the dosage of two solutions, one acidic and one basic, and by means of a feedback control based on the pH value measured by a probe, it is possible to simulate *in vitro* pH "histories" similar to those occurring *in vivo,* both with decreasing pH and with increasing pH, thus being able to obtain release data of active ingredients more similar to the real ones.

The device takes from two different tanks, by means of pumps, for example peristaltic pumps, a basic or an acid solution having have the purpose of correcting the pH to follow a reference value (set point) which varies over time. Non-limiting examples of such solutions can be distilled water and sodium hydroxide, and distilled water and hydrochloric acid. The proposed device, in addition to the verification and feedback control of the pH of the aforementioned solution, allows accurate monitoring, instant by instant, of the volumes involved, thanks to an innovative system for determining the flow rate of the metering pumps (for example peristaltic), which allows to avoid the addition of flow meters in the device.

In the light of the current limitations of *in vitro* tests, the present invention, therefore, confers greater reliability to said tests, guaranteeing the simulation of pH "histories" comparable to those that are carried out in vivo.

## Brief description of the drawings

The specific features of the present invention, as well as its advantages, will be more apparent with reference to the attached figures, in which:

Figure 1 shows a process flowsheet (PFD: Process Flow Diagram) of the device according to the invention (in the dashed box) in the preferred operating configuration;
Figure 2 shows an engineering flowsheet (P&ID: Piping and Instrumentation Diagram) of the device of Figure 1 (in the dashed box) in the preferred operating configuration;

**Figures 3a** and **3b** respectively show an exploded perspective view of one of the two peristaltic pumps present in a preferred embodiment of the device of the invention, and a perspective view of the same pump evidencing the installation of a Hall effect sensor on the pump head itself;

**Figure 4** shows an example of pH control for the simulation of postprandial conditions; on the left the set-point pH and that measured in the release medium, on the right the total volume of solution, acid or basic, added;

**Figure 5** shows an example of pH control for a generic pH function which provides for a descending acid ramp followed by a positive step; on the left the set-point pH and that measured in the release medium, on the right the total volume of solution, acid or basic, added; and

**Figure 6** shows an example of pH control for a generic pH function which involves a double step, one negative and the other positive; on the left the set-point pH and that measured in the release medium, on the right the total volume of solution, acid or basic, added.

**Detailed description of the invention**

The present invention specifically provides a method for the measurement and feedback control of the pH of a saline buffer solution, in which device set points are followed, describing the evolution of physiological pH which takes place during the phases of digestion in the stomach. The method developed includes the following operations:

- calibration for the pH reading, through the use of solutions at known pH, for example 4 and 7, for the detection of hydrogen ion concentration in the solution that constitutes the release medium, and subsequent correlation with the electric potential values provided by a pH probe, to obtain a calibration line;
- preparation of pH correction solutions using acidic and basic aqueous solutions, which may consist of aqueous solutions of inorganic acids and bases, such as hydrochloric acid and sodium hydroxide, or of organic acids and bases, or biological fluids or simulated biological fluids;
- periodic reading of the pH of the release solution by means of a specific probe;
- periodic comparison of the measured pH value with the pH set-point value, variable over the set period of time;
- periodic feedback control action, resulting in an addition, to said release medium, of acid or basic correction solution, to minimize the difference between the desired set-point value and value;
- periodic quantification of the volume of correction solution added.

This last operation is essential for calculating the percentage of drug released moment by moment, as the introduction of pH corrective solutions, acid or basic, alters the volume of the release medium. The calculation of the percentage of drug released passes through the quantification of the active ingredient through, for example, UV-VIS spectrophotometry or similar analytical techniques, which have as a response the concentration of the active ingredient in solution, and the precise knowledge of the volume of the release means is therefore necessary to quantify the mass of drug released, knowing its concentration, and then to calculate the percentage of drug released.

The quantification of the added volume can be performed by monitoring and/or setting the operating parameters of the pump used for the addition of the correcting solution during the control action, or by using volumetric flow meters.

In the event that the pump operating parameters are monitored and/or set, a first calibration phase of the pumps may be necessary, in which the volumetric flow rate can be correlated to the parameters of the pump themselves, which can be measured and/or modulated, such as the number of revolutions of the motor, the supply voltage, the number of revolutions of the head in peristaltic pumps, etc.. In this way, by measuring or imposing these parameters in a given control action of the system, it is possible to know the quantity of solution delivered without having to resort to direct flow meters.

Once the relationship between the characteristic parameters of the pump used and its volumetric flow rate is known, it is possible to measure the quantity of acidic or basic solution dispensed instant by instant.

According to a further aspect thereof, the present invention concerns a device for the measurement and the feedback control of the pH of a buffer solution representing the release medium in a device for the dissolution and release testing of pharmaceutical formulations for oral administration, in which device set points are followed that describe the evolution of physiological pH occurring during the phases of digestion in the stomach. This device can be equipped with two tanks, preferably of a volume not exceeding 1 L, one for the storage of the acidic solution and one for the basic one; two pumps, preferably peristaltic pumps, each connected with the suction port to one of the two tanks and with the delivery port to the tank in which the release test is carried out, such as for example a beaker or a "vessel" in standard devices of the USP II type; a pH sensor; sensors for monitoring the volume of acid and basic correction solution added.

The device can be equipped with direct flow meters or with indirect meters, which are based on the measurement of the operating parameters of the pump. The electronics of the device can be managed by a microcontroller in charge of both the acquisition of the signals of the various sensors and the implementation of the control logic, using a specific algorithm. In fact, having access to analog and digital ports, the microcontroller is able to acquire the various types of signals, calculate the necessary feedback control action and drive, generating output signals, one or more controllers driving the pumps. Everything can be equipped with a screen, such as LCD or OLED screen, and keys for stand-alone applications.

The device according to the present invention is able, by implementing a feedback control, for example of the proportional (P), proportional integral (PI) or proportional integral derivative (PID) type, to regulate the delivery time of the peristaltic pumps in order to have the minimum deviation from the set-point pH value set for a generic instant. Therefore, in the device, the signal read by the pH probe is usually acquired as an analog signal (voltage) by the microcontroller, is compared with the predetermined pH value (set-point) at a certain time and, by means of a control algorithm, the acid or basic solution pump is activated for a given period of time.

Thanks to the use of sensors for the direct quantification of the volumetric flow rate and/or for monitoring the operating parameters of the pump, it is possible to detect instant by instant the volume of acid or basic correction solution added.

In a preferred embodiment of the device of the invention, peristaltic pumps are used, in which the control of the number of revolutions of the pump is carried out on the head and not on the motor, using Hall effect sensors and magnetic disks embedded in the rollers present in the head of the pump. In this way, it is possible to use different types of peristaltic pumps, even the particularly economical ones, in which the number of head revolutions is not directly proportional or in any case not directly correlated to the number of motor revolutions, obtaining reliable measurements of the volumetric flow rate.

This procedure, carried out at predetermined time intervals, for example every 10 seconds, allows to control the pH of the buffer solution constituting the release medium so as to simulate *in vitro* pH "histories" (i.e., evolutions) which usually occur *in vivo*.

In many models of peristaltic dosing pumps the motor-rollers coupling is not fixed, and this means that the number of motor revolutions is not equal to the number of revolutions of the rollers. In order to measure the pump flow rate (or the volume delivered in a certain operating time) it is necessary, therefore, to measure and relate, by means of a calibration, the number of revolutions of the rollers in the pump head with the volumetric flow rate. For this purpose, according to a preferred embodiment of the invention, a measurement system based on the magnetic field generated by a set of neodymium magnets, and the quantification of said number of revolutions by using Hall effect sensors has been created.

In particular, cylindrical neodymium magnets suitable for being inserted inside the rollers of a peristaltic pump, for example having a diameter of 3 mm and a thickness of 0.5 mm, are stacked, for example forming a set of 5 magnets to increase the intensity of the magnetic field generated. This set is housed inside the one or more rollers present in the pump head. Outside the peristaltic pump, a Hall effect sensor is installed by gluing or using special supports. As the stack of magnets passes during the revolution, the Hall effect sensor will be "activated", thus returning a pulse signal, usually digital in DC, of a "high" type (for example 5V or 3.3V). By using a microcontroller it is possible to count the pulses returned in the unit of time, and therefore to measure the number of passes of the rollers, below the Hall effect sensor, in the unit of time. It is therefore possible to quantify, having known the amount of rollers "loaded" with the magnets and the number of pulses per unit of time, the revolutions per minute (rpm) performed by the pump rollers.

Since in peristaltic pumps the number of revolutions of the rollers is directly proportional to the delivered volumetric flow rate, it is thus possible to construct a calibration curve by measuring the delivered volumetric flow, when the number of revolutions of the pump is known. In this way it will be possible, during use, to exploit the calibration curve to quantify the volume delivered by the pump, knowing the number of revolutions made by the rollers in the pump head.

**EXAMPLES**

Some specific embodiments of the method and of the device for controlling the pH in conventional devices for the delivery of drugs, according to the invention, are described hereafter by way of example but not of limitation, together with the results of the experiments carried out.

**Process Flow and Piping and Instrumentation Diagrams**

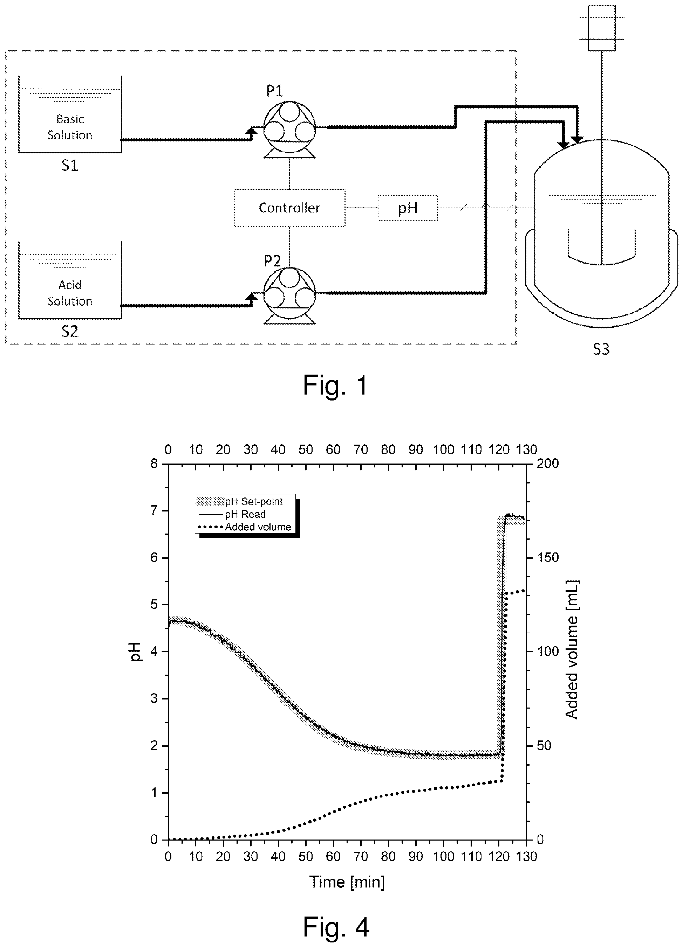

A process flowsheet for realizing the device of the present invention is shown in **Figure 1.**

To carry out the method of the invention, a basic aqueous solution, for example 1 M NaOH, and an acidic aqueous solution, for example 1 M HCl, are respectively placed in the tanks S1 and S2. These tanks are connected by piping, for example silicone or PVC flexible pipes, to the suction port of the peristaltic pumps, P1 and P2, whose delivery port is connected by another pipe to the tank S3 in which the release tests are carried out: a thermostated beaker provided with stirrer or a standardized apparatus, example USP II. The appropriately calibrated pH probe is inserted in the tank S3, which communicates with a controller which activates the base pump (P1) or the acid pump (P2) in proportion to the difference between the set-point pH value and the measured pH value.

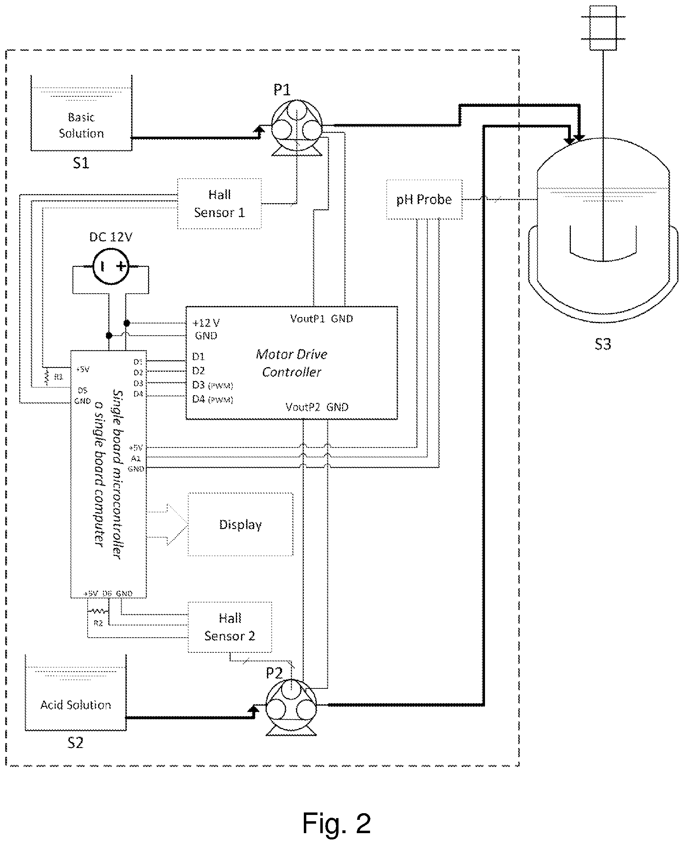

An engineering flowsheet diagram (also known as P&ID: Piping and Instrumentation Diagram) is shown in **Figure 2.** The device in charge of the calculation, a single board microcontroller, powered by direct current at 12 V, is also used to control the instrumentation of the device through the use of a Motor Drive Controller, such as an L298N, also powered by direct current at 12 V. It is thus possible to control the switching on and off of the peristaltic pumps P1 and P2. The signal of the pH sensor, placed in the tank S3, is transduced in a voltage difference and read on an analog port by the microcontroller. In the microcontroller, the voltage read is converted into a pH value by exploiting the calibration line constructed through a specific procedure and code. This pH value measured at a generic time is compared by the microcontroller itself with the preset time-dependent pH value (set-point pH). If the measured pH is lower than the set-point, the microcontroller, through the "Motor Drive Controller", activates the pump of the base P1, vice versa the acid pump P2. The running time of the pumps can be proportional to the difference between the measured value and the set-point value.

The diagram of Figure 2 also shows the presence of a display which, associated with the presence of selectors (keys) already present on the micro controller or specially inserted (not shown in Figure 2), result in a stand-alone. device.

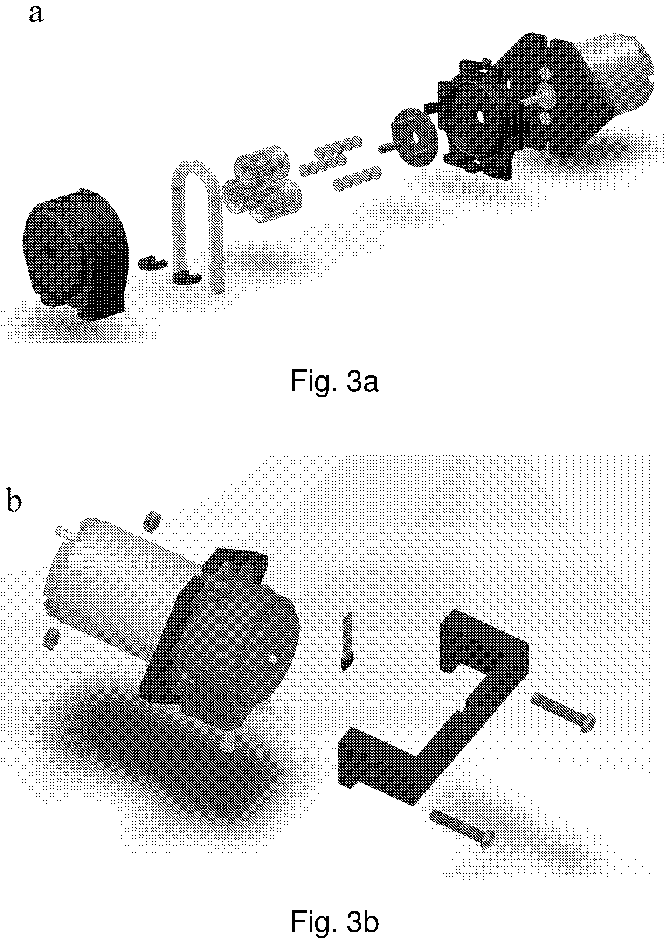

**Figures 3a** and **3b** show a schematic diagram of the modifications made, according to a preferred embodiment of the device of the invention, to realize a system for controlling the revolutions of the rollers in a peristaltic pump based on the use of neodymium discs and a Hall effect sensor. These changes provide for the insertion of neodymium magnets embedded in the pump rollers, as well as the installation, using a specially designed support, built by fused deposition modeling (FDM), for the Hall effect sensor.

In particular, Figure 3a shows an exploded view of the pump head in the rollers of which 5 neodymium magnets are inserted, stacked. Figure 3b shows an exploded view showing the installation of the Hall effect sensor on the pump head using a special support designed and printed in 3D.

During the operation of the pumps, the Hall effect sensors mounted on the heads of the pumps measure the actual revolutions of the pump by means of the magnetic field generated by the magnetic discs, for example neodymium magnets, inserted inside the pump rollers. The Hall effect sensor, with digital output and presence of pull-up resistors (R1 and R2), provides a high level signal ("1") when the roller (containing the magnets) of the peristaltic pump is aligned with the sensor and a low level signal ("0") when the roller is not aligned with the sensor. When the pump is rotating, the Hall effect sensor records the number of roller passes, which can easily be converted into the pump head rpm number.

Having previously estimated, by constructing a calibration curve, and implemented on the microcontroller, the relationship between rpm and volumetric flow rate of the pump, it is possible to precisely quantify the volume delivered by the pump at the i-th pH correction.

**Examples of pH control in standard USP II apparatus**

**Figures 4, 5** and **6** show examples of pH control by using the method and device object of the present invention, applied to the vessel of a USP II device operated at 37°C, at 100 rpm with paddle stirrer. On the left, in red, the set-point pH and the pH measured in the release medium are shown, while on the right the total volume of solution, acid or basic, added to make the necessary pH changes is shown.

The tests shown in the aforementioned figures were carried out using an aqueous solution of 1 M HCl as acid solution and an aqueous solution of 1 M NaOH as a basic solution. The release medium whose pH was checked consisted of 0.5 L of a buffer solution obtained by mixing 38 g of tribasic phosphate dodecahydrate and 16.6 mL of 37% w/w HCl in distilled water.

In particular, **Figure 4** shows an example of pH control for simulating postprandial conditions recorded in vivo. The initial pH, equal to 4.6, is typical of immediately postprandial conditions and was obtained *in vitro* using a buffer solution produced by mixing 38 g of tri-basic phosphate dodecahydrate and 16.6 mL of 37% w/w HCl in water distilled. As reported in the literature, the acidity of gastric fluids gradually decreases until reaching a pH of 1.8 2 hours after the end of the meal. After this time, due to the passage of material (food and/or drugs) from the stomach to the intestine, the pH drastically changes, reaching a value of 6.8. The pH set-point, as a function of time, was described by means of a polynomial of the 9th degree for the first 120 minutes, and with a constant value of 6.8 for the subsequent instants.

$$pH_{set-point}(t)$$

$$= \begin{cases} a_1 + a_2 t + a_3 t^2 + a_4 t^3 + a_5 t^4 + a_6 t^5 + a_7 t^6 + a_8 t^7 + a_9 t^8 + a_{10} t^9 & t \leq 120\ min \\ 6.8, & t > 120\ min \end{cases}$$

**Table 1. Coefficients of the equation used to simulate the post-prandial pH evolution**

| $a_1$ | $a_2$ | $a_3$ | $a_4$ | $a_5$ | $a_6$ | $a_7$ | $a_8$ | $a_9$ | $a_{10}$ |
|---|---|---|---|---|---|---|---|---|---|
| 4.66684 | 0.00887 | -0.00323 | $2.02*10^{-6}$ | $-9.5*10^{-6}$ | $2.46*10^{-7}$ | $-3.5*10^{-9}$ | $2.87*10^{-11}$ | $-1.2*10^{-13}$ | $2.19*10^{-16}$ |

As it can be seen, the measured pH faithfully describes the set pH, thus demonstrating the validity of the proposed method and device.

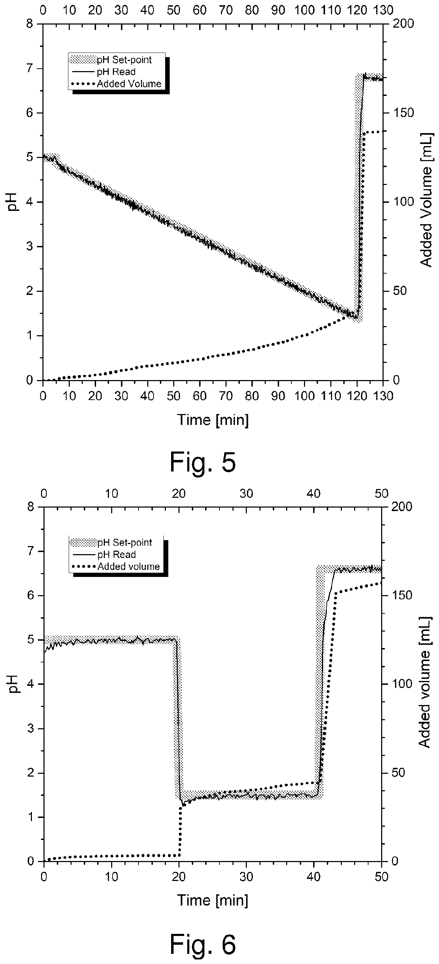

**Figure 5** and **Figure 6** show the results obtained by implementing generic pH set-point functions in order to demonstrate the flexibility of the proposed method and device. As in the previous case, the acid solution consisted of 1 M HCl and the basic solution of 1 M NaOH. The release medium was obtained by dissolving 9 g of PBS in 0.5 L of deionized water, to which a volume of 7.2 mL HCl 37% w/w was added to reach the initial pH conditions.

In particular, Figure 5 shows the result in terms of pH control and total volume of acidic and basic solution dosed for a set-point pH constructed as a stationary phase at pH 5 followed by a descending ramp and a positive step, which can be described using the following function:

$$pH_{set-point}(t) = \begin{cases} 5, & t \leq 5\,min \\ pH = -0.032t + 5.065, & 5 < t \leq 120\,min \\ 6.8, & t > 120\,min \end{cases}$$

It should be noted the a limited volume is used, as in the previous case, of acidic and basic solutions for times less than 2 hours, thanks to the use of particularly concentrated solutions, and a well-balanced feedback control.

Figure 6 shows the result, in terms of pH control and total volume of acid and basic solution dosed, for a set-point pH consisting of 3 stationary phases, i.e. a first phase at pH 5, followed by a second phase at pH 1.5 and a third phase at pH 6.8, which can be described using the function:

$$pH_{set-point}(t) = \begin{cases} 5, & t \leq 20\,min \\ 1.5, & 20 < t \leq 40\,min \\ 6.6, & t > 40\,min \end{cases}$$

In conclusion, the device proposed according to the invention is capable of operating on its own in an independent manner (stand-alone), analyzing and immediately correcting, by using two easy-to-prepare solutions, a sensor of pH and a feedback loop control system, the pH value of an aqueous environment, even buffered.

**Claims**

1. A process for the measurement and feedback control of the pH of a buffer solution forming the release medium in a device for testing the dissolution and release of pharmaceutical formulations for oral administration, in which device set points are followed, reproducing the evolution of physiological pH which takes place in the course of digestion in the stomach, which process includes the following operations:

   a) calibration for the pH reading, through the use of solutions at known pH, for example 4 and 7, for the detection of hydrogen ion concentration in the release medium, and subsequent correlation with the electric potential values provided by a pH probe, to obtain a calibration line;
   b) preparation of pH correction solutions using acid and basic aqueous solutions, consisting of aqueous solutions of inorganic or organic acids and bases, or biological fluids, or simulated biological fluids;
   c) periodic reading of the pH of the release solution by means of a pH probe;
   d) periodic comparison of the pH value measured with the pH set-point, variable over the set period of time;
   e) periodic feedback control action, resulting in an addition, to said release medium, of an acid or basic correction solution, to minimize the difference between the desired set-point value and the measured value;
   f) periodic quantification of the volume of correction solution added, said quantification being used to determine the drug concentration in the release medium.

2. The process for the measurement and feedback control of pH according to claim 1, wherein said pH correction solutions of operation b) are aqueous hydrochloric acid and aqueous sodium hydroxide solutions.

3. The process for the measurement and feedback control of pH according to claims 1 or 2, wherein the time interval at which said periodic operations c) - f) are carried out is comprised between 2 and 10 seconds.

4. The process for the measurement and feedback control of pH according to claim 3, wherein said time interval is 5 seconds.

5. The process for the measurement and feedback control of pH according to any one of claims 1-4, wherein said periodic quantification f) of the volume of correction solution added to the release medium is obtained by determining the volumetric flow rate of said correction solutions.

6. The process for the measurement and feedback control of pH according to any one of claims 1-4, wherein said periodic quantification f) of the volume of correction solution added to the release medium is obtained by detecting or setting an operational parameter related to a positive displacement pump (P1, P2) which feeds the corresponding correction solution.

7. The process for the measurement and feedback control of pH according to claim 6, wherein said positive displacement pump (P1, P2) is a peristaltic pump, and said detected parameter is the number of revolutions of its head.

8. The process for the measurement and feedback control of pH according to claim 7, wherein said number of revolutions is detected by means of Hall-effect sensors and magnetic elements embedded in one or more rotating elements present in the head of the pump (P1, P2).

9. A device for the measurement and feedback control of the pH of a buffer solution forming the release medium in a device for the dissolution and release of pharmaceutical formulations for oral administration, in which set points are followed, reproducing the evolution of physiological pH which takes place in the course of digestion in the stomach, which device includes:

   two tanks (S1, S2), preferably of a volume not exceeding 1 L, for the storage of two pH correction solutions, namely an acid solution and a basic solution;
   two pumps (P1, P2), preferably peristaltic pumps, each connected at the suction side to one of said two tanks (S1, S2), and both connected at their discharge side to an external tank (S3) where dissolution and release test of pharmaceutical formulations is carried out, the head of each one of said pumps (P1, P2) being provided, within one of more of the rollers which represent the system for displacing the respective solution, with one or more disks or cylinders of a magnetic material;
   a pH sensor connected to said external tank (S3);
   two sensors configured to indirectly monitor the volume of added acid and basic correction solution in a time unit, which sensors consist of two Hall-effect sensors, each fixedly connected to the head of the respective pump and configured to cooperate with said magnetic disks or cylinders placed within said peristaltic pumps to quantify the number of revolutions of said pumps in a time unit, and hence the volume rate of acid or basic solution delivered, by means of the use of a previously determined calibration line;
   a feedback control system configured to drive one or the other of said two pumps, depending on the pH value detected in said external tank and on a set-point value set for a generic instant;
   a microcontroller configured to control both the acquisition of signals from the various sensors and the implementation of the control logic, by means of a specific algorithm.

10. The device for the measurement and feedback control of the pH according to claim 9, wherein said one or more disks or cylinders of magnetic material are neodymium magnets.

11. The device for the measurement and feedback control of the pH according to claims 9 or 10, wherein each of said peristaltic pumps (P1, P2) comprises three rollers, and within each one of said rollers five neodymium magnetic disks are stacked.

12. The device for the measurement and feedback control of pH according to any one of claims 9-11, also comprising a screen, with keys for the stand-alone working of the device itself.

**Patentansprüche**

1. Verfahren zur Messung und Rückkopplungsregelung des pH-Werts einer Pufferlösung, die das Freisetzungsmedium in einem Gerät zum Testen der Auflösung und Freisetzung pharmazeutischer Formulierungen zur oralen Verabreichung bildet, bei dem Gerätesollwerte befolgt werden, um die Entwicklung zu reproduzieren des physiologischen pH-Wertes, der im Verlauf der Verdauung im Magen stattfindet, wobei das Verfahren die folgenden Schritte umfasst:

   a) Kalibrierung des pH-Werts durch Verwendung von Lösungen mit bekanntem pH-Wert, beispielsweise 4 und 7, zur Erkennung der Wasserstoffionenkonzentration im Freisetzungsmedium und anschließende Korrelation mit den von a bereitgestellten elektrischen Potentialwerten pH-Sonde, um eine Kalibrierungslinie zu erhalten;
   b) Herstellung von pH-Korrekturlösungen unter Verwendung saurer und basischer wässriger Lösungen, bestehend aus wässrigen Lösungen anorganischer oder organischer Säuren und Basen, oder biologischen Flüssigkeiten oder simulierten biologischen Flüssigkeiten;
   c) periodisches Ablesen des pH-Wertes der freigesetzten Lösung mittels einer pH-Sonde;
   d) periodischer Vergleich des gemessenen pH-Wertes mit dem pH-Sollwert, der über den eingestellten Zeitraum variabel ist;
   e) periodische Rückkopplungsregelung, die zu einer Zugabe einer sauren oder basischen Korrekturlösung zu dem Freisetzungsmedium führt, um die Differenz zwischen dem gewünschten Sollwert und dem gemessenen Wert zu minimieren;
   f) periodische Quantifizierung des Volumens der zugesetzten Korrekturlösung, wobei diese Quantifizierung zur Bestimmung der Wirkstoffkonzentration im Freisetzungsmedium verwendet wird.

2. Verfahren zur Messung und Rückkopplungsregelung des pH-Werts nach Anspruch 1, wobei die pH-Korrekturlösungen von Schritt b) wässrige Salzsäure- und wässrige Natriumhydroxidlösungen sind.

3. Verfahren zur Messung und Rückkopplungsregelung des pH-Werts gemäß Anspruch 1 oder 2, wobei das Zeitintervall, in dem die periodischen Vorgänge c)-f) ausgeführt werden, zwischen 2 und 10 Sekunden beträgt.

4. Verfahren zur Messung und Rückkopplungsregelung des pH-Werts nach Anspruch 3, wobei das Zeitintervall 5 Sekunden beträgt.

5. Verfahren zur Messung und Rückkopplungsregelung des pH-Werts nach einem der Ansprüche 1 bis 4, wobei die periodische Quantifizierung f) des Volumens der dem Freisetzungsmedium zugesetzten Korrekturlösung durch Ermittlung der Volumenstromrate der Korrekturlösungen erfolgt.

6. Verfahren zur Messung und Rückkopplungsregelung des pH-Werts nach einem der Ansprüche 1 bis 4, wobei die periodische Quantifizierung f) des Volumens der dem Freigabemedium zugesetzten Korrekturlösung durch Erfassen oder Einstellen eines Betriebsparameters in Bezug auf eine Verdrängerpumpe (P1, P2) erfolgt, die die entsprechende Korrekturlösung zuführt.

7. Verfahren zur Messung und Rückkopplungsregelung des pH-Werts nach Anspruch 6, wobei die Verdrängerpumpe (P1, P2) eine Schlauchpumpe ist und der erfasste Parameter die Anzahl der Umdrehungen ihres Kopfes ist.

8. Verfahren zur Messung und Rückkopplungsregelung des pH-Werts nach Anspruch 7, wobei die Anzahl der Umdrehungen mittels Hall-Effekt-Sensoren und magnetischen Elementen erfasst wird, die in einem oder mehreren rotierenden Elementen im Kopf der Pumpe (P1, P2) eingebettet sind.

9. Vorrichtung zur Messung und Rückkopplungsregelung des pH-Werts einer Pufferlösung, die das Freisetzungsmedium in einer Vorrichtung zur Auflösung und Freisetzung pharmazeutischer Formulierungen zur oralen Verabreichung bildet, in der Sollwerte eingehalten werden, die die Entwicklung des physiologischen pH-Werts reproduzieren, die im Verlauf der Verdauung im Magen stattfindet, wobei die Vorrichtung umfasst:

   zwei Tanks (S1, S2), vorzugsweise mit einem Volumen von höchstens 1 l, zur Lagerung von zwei pH-Korrekturlösungen, nämlich einer Säurelösung und einer basischen Lösung;
   zwei Pumpen (P1, P2), vorzugsweise Schlauchpumpen, die jeweils an der Saugseite mit einem der beiden Tanks (S1, S2) verbunden sind und beide an ihrer Druckseite mit einem externen Tank (S3) verbunden sind, in dem Auflösungs- und Freisetzungstests pharmazeutischer Formulierungen durchgeführt werden, wobei der Kopf jeder der Pumpen (P1, P2) innerhalb einer oder mehrerer der Rollen, die das System zum Verdrängen der jeweiligen Lösung darstellen, aus einer oder mehreren Scheiben oder Zylindern aus einem magnetischen Material besteht;

ein pH-Sensor, der mit dem externen Tank (S3) verbunden ist;

zwei Sensoren zur indirekten Überwachung des Volumens der hinzugefügten Säure- und Basenkorrekturlösung in einer Zeiteinheit, wobei die Sensoren aus zwei Hall-Effekt-Sensoren bestehen, die jeweils fest mit dem Kopf der jeweiligen Pumpe verbunden sind und mit den in den Schlauchpumpen platzierten Magnetplatten oder Zylindern zusammenarbeiten, um die Anzahl der Umdrehungen der Pumpen in einer Zeiteinheit und damit die Volumenrate der gelieferten Säure- oder Basenlösung mithilfe einer zuvor festgelegten Kalibrierungslinie zu quantifizieren;

ein Rückkopplungsregelungssystem, das je nach dem im externen Tank erfassten pH-Wert und einem für einen allgemeinen Zeitpunkt festgelegten Sollwert die eine oder andere der beiden Pumpen antreibt;

ein Mikrocontroller, der sowohl die Erfassung der Signale von den verschiedenen Sensoren als auch die Implementierung der Steuerlogik mithilfe eines spezifischen Algorithmus steuert.

10. Vorrichtung zur Messung und Rückkopplungsregelung des pH-Werts nach Anspruch 9, wobei die eine oder mehreren Scheiben oder Zylinder aus magnetischem Material Neodym-Magnete sind.

11. Vorrichtung zur Messung und Rückkopplungsregelung des pH-Werts nach Anspruch 9 oder 10, wobei jede der Schlauchpumpen (P1, P2) drei Rollen umfasst und in jeder dieser Rollen fünf Neodym-Magnetscheiben gestapelt sind.

12. Vorrichtung zur Messung und Rückkopplungsregelung des pH-Werts gemäß einem der Ansprüche 9 bis 11, das außerdem einen Bildschirm mit Tasten für den eigenständigen Betrieb des Geräts selbst umfasst.

**Revendications**

1. Procédé de mesure et de rétrocontrôle du pH d'une solution tampon formant le milieu de libération dans un dispositif de test de dissolution et de libération de formulations pharmaceutiques pour administration orale, dans lequel on suit les consignes du dispositif reproduisant l'évolution du pH physiologique qui s'effectue au cours de la digestion dans l'estomac, lequel procédé comprend les opérations suivantes:

a) étalonnage de la lecture du pH, grâce à l'utilisation de solutions à pH connu, par exemple 4 et 7, pour la détection de la concentration en ions hydrogène dans le milieu de libération, et corrélation ultérieure avec les valeurs de potentiel électrique fournies par une sonde de pH, pour obtenir une ligne d'étalonnage;

b) préparation de solutions de correction du pH à l'aide de solutions aqueuses acides et basiques, constituées de solutions aqueuses d'acides et de bases inorganiques ou organiques, ou de fluides biologiques, ou de fluides biologiques simulés;

c) lecture périodique du pH de la solution de libération au moyen d'une sonde de pH;

d) comparaison périodique de la valeur du pH mesurée avec le point de consigne du pH, variable sur la période de temps définie;

e) action de rétroaction périodique, aboutissant à l'ajout, audit milieu de libération, d'une solution de correction acide ou basique, pour minimiser la différence entre la valeur de consigne souhaitée et la valeur mesurée;

f) quantification périodique du volume de solution correctrice ajoutée, ladite quantification étant utilisée pour déterminer la concentration en médicament dans le milieu de libération.

2. Procédé de mesure et de rétrocontrôle du pH selon la revendication 1, dans lequel lesdites solutions de correction du pH de l'opération b) sont des solutions aqueuses d'acide chlorhydrique et des solutions aqueuses d'hydroxyde de sodium.

3. Procédé de mesure et de rétrocontrôle du pH selon les revendications 1 ou 2, dans lequel l'intervalle de temps pendant lequel lesdites opérations périodiques c) - f) sont effectuées est compris entre 2 et 10 secondes.

4. Procédé de mesure et de rétrocontrôle du pH selon la revendication 3, **caractérisé en ce que** ledit intervalle de temps est de 5 secondes.

5. Procédé de mesure et de rétrocontrôle du pH selon l'une quelconque des revendications 1 à 4, dans lequel ladite quantification périodique f) du volume de solution de correction ajoutée au milieu de libération est obtenue en déterminant le débit volumétrique de ladite solution de correction. solutions.

6. Procédé de mesure et de rétrocontrôle du pH selon l'une quelconque des revendications 1 à 4, dans lequel ladite quantification périodique f) du volume de solution de correction ajoutée au milieu de libération est obtenue en détectant ou en définissant un paramètre opérationnel lié à une pompe volumétrique (P1, P2) qui alimente la solution de correction correspondante.

7. Procédé de mesure et de rétrocontrôle du pH selon la revendication 6, dans lequel ladite pompe volumétrique (P1, P2) est une pompe péristaltique, et ledit paramètre détecté est le nombre de tours de sa tête.

8. Procédé de mesure et de rétrocontrôle du pH selon la revendication 7, dans lequel ledit nombre de tours est détecté au moyen de capteurs à effet Hall et d'éléments magnétiques noyés dans un ou plusieurs éléments rotatifs présents dans la tête de pompe (P1 ,P2).

9. Dispositif de mesure et de rétrocontrôle du pH d'une solution tampon formant le milieu de libération dans un dispositif de dissolution et de libération de formulations pharmaceutiques pour administration orale, dans lequel sont suivies des valeurs de consigne reproduisant l'évolution du pH physiologique qui a lieu au cours de la digestion dans l'estomac, lequel dispositif comprend :

   deux réservoirs (S1, S2), de préférence d'un volume n'excédant pas 1 L, pour le stockage de deux solutions de correction de pH, à savoir une solution acide et une solution basique ;
   deux pompes (P1, P2), de préférence péristaltiques, chacune reliée du côté aspiration à l'un desdits deux réservoirs (S1, S2), et toutes deux reliées du côté refoulement à un réservoir externe (S3) où se déroulent la dissolution et la libération on effectue un test de formulations pharmaceutiques, la tête de chacune desdites pompes (P1, P2) étant munie, à l'intérieur d'un ou plusieurs des rouleaux qui représentent le système de déplacement de la solution respective, d'un ou plusieurs disques ou cylindres d'un matériau magnétique;
   un capteur de pH connecté audit réservoir externe (S3) ;
   deux capteurs pour surveiller indirectement le volume de solution de correction acide et basique ajoutée dans une unité de temps, lesquels capteurs sont constitués de deux capteurs à effet Hall, chacun relié de manière fixe à la tête de la pompe respective et coopérant avec lesdits disques ou cylindres magnétiques placés à l'intérieur dudit des pompes péristaltiques pour quantifier le nombre de tours desdites pompes dans une unité de temps, et donc le débit volumique de solution acide ou basique délivré, au moyen de l'utilisation d'une ligne d'étalonnage préalablement déterminée ;
   un système de asservissement qui pilote l'une ou l'autre desdites deux pompes, en fonction de la valeur du pH détectée dans ledit réservoir externe et d'une valeur de consigne fixée pour un instant générique ;
   un microcontrôleur, contrôlant à la fois l'acquisition des signaux des différents capteurs et la mise en oeuvre de la logique de contrôle, au moyen d'un algorithme spécifique.

10. Dispositif de mesure et de rétrocontrôle du pH selon la revendication 9, dans lequel lesdits un ou plusieurs disques ou cylindres en matériau magnétique sont des aimants en néodyme.

11. Dispositif de mesure et de rétrocontrôle du pH selon les revendications 9 ou 10, dans lequel chacune desdites pompes péristaltiques (P1, P2) comprend trois rouleaux, et à l'intérieur de chacun desdits rouleaux cinq disques magnétiques en néodyme sont empilés.

12. Dispositif de mesure et de contrôle rétroactif du pH selon l'une quelconque des revendications 9 à 11, comprenant également un écran, avec des touches pour le fonctionnement autonome du dispositif lui-même.

Fig. 1

Fig. 4

Fig. 2

a

Fig. 3a

b

Fig. 3b

Fig. 5

Fig. 6

## REFERENCES CITED IN THE DESCRIPTION

### Patent documents cited in the description

- CN 108364553 B

### Non-patent literature cited in the description

- **DRESSMAN, J.B. et al.** Upper Gastrointestinal (GI) pH in Young, Healthy Men and Women. *Pharm. Res.,* 1990, vol. 7 (7), 756-761
- *The United States Pharmacopeial Convention (USP), Harmonization methods,* 2011, hiips://www usp org/sites/default/files/usp/documen t/har-monization/gen-method/stage_6_monograph_25_feb_2011.pdf
- InfoGest consensus method. **MACKIE, A. ; RIGBY, N.** The Impact of Food Bioactives on Health. Springer, 2015, 13-22
- **BRODKORB, A. et al.** INFOGEST static in vitro simulation of gastrointestinal food digestion. *Nature Protocols,* 2019, vol. 14 (4), 991-1014
- **MULET-CABERO, A.-I. et al.** A standardized semi-dynamic in vitro digestion method suitable for food - an international consensus. *Food Funct.,* 2020, vol. 11 (2), 1702-1720
- **VATIER, J. et al.** A model of an 'artificial stomach' for assessing the characteristics of an antacid. *Aliment. Pharmacol. Therap.,* 1988, vol. 2 (5), 461-470
- **KONG, F. ; SINGH, R.P.** A human gastric simulator (HGS) to study food digestion in human stomach. *J. Food Sci.,* 2010, vol. 75 (9), E627-E635
- Dynamic gastric model (DGM). **THUENEMANN, E.C. et al.** The Impact of Food Bioactives on Health. Springer, 2015, 47-59
- **MINEKUS, M.** The TNO gastro-intestinal model (TIM). *The Impact of Food Bioactives on Health,* 2015, 37-46
- **MÉNARD, O. et al.** Validation of a new in vitro dynamic system to simulate infant digestion. *Food Chem.,* 2014, vol. 145, 1039-1045
- **CASCONE, S. et al.** In vitro simulation of human digestion: chemical and mechanical behavior. *Dissolution Technol.,* 2016, vol. 23 (4), 16-23